# EUROPEAN PATENT APPLICATION

(11) **EP 4 635 495 A1**
(43) Date of publication of application: **22.10.2025**
(21) Application number: 22968123.4
(22) Date of filing: 13.12.2022
(51) Int. Cl.: A61K 31/437, A61K 31/416, C07D 231/56, C07D 471/04, A61P 27/02

(54) **USE OF NAPHTHYLUREA COMPOUNDS IN PREPARATION OF DRUGS FOR TREATING PTERYGIUM**

(71) Applicant: Suzhou Raymon Pharmaceuticals Company, Ltd., Suzhou, Jiangsu 215123 (CN)
(72) Inventor: ZHANG, Fei, Suzhou, Jiangsu 215123 (CN); ZHANG, Yanhong, Suzhou, Jiangsu 215123 (CN); WANG, Sen, Suzhou, Jiangsu 215123 (CN)
(74) Representative: Henderson, Helen Lee
(86) International application number: PCT/CN2022/138801
(87) International publication number: WO 2024/124411

(57) **Abstract**

The use of naphthylurea compounds in preparation of drugs for treating pterygium. The present invention particularly relates to the use of substances A in the preparation of drugs, the substances A being naphthylurea compounds represented by formula I, or pharmaceutically acceptable salts thereof, solvates thereof, solvates of said pharmaceutically acceptable salts, crystal forms thereof or tautomers thereof, the drugs being used for pterygium.

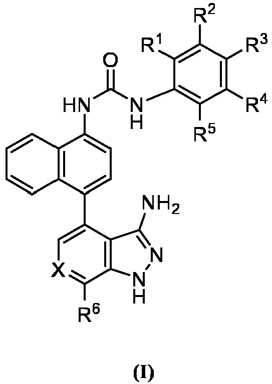

## Description

### TECHNICAL FIELD

The present disclosure relates to a use of a naphthylurea compound in the manufacture of a medicament for the treatment of pterygium.

### BACKGROUND

Pterygium, also known as "surfer's eye", usually manifests in the palpebral fissure area with thickened conjunctiva, and the fibrous vascular tissue of bulbar conjunctiva is thickened and grows in a triangular shape. The head of the pterygium grows from the lateral canthus to the medial canthus, gradually increasing in size and invading into the corneal tissue. At the same time, the dilated fibrous blood vessels also grow to the central part of the cornea. The excessively proliferated and hypertrophied pterygium tissue can even cover the entire pupil area, affecting the normal movement of the eyeballs, is a kind of ophthalmic disease. After the onset of the disease, the patient manifests in reduction in visual acuity, foreign body sensation within the eye and occlusion of light, thereby affecting vision. Currently, the main clinical method for treating pterygium is the surgical excision method, but there is a high recurrence rate after surgery.

### CONTENT OF THE PRESENT INVENTION

The technical problem to be solved by the present disclosure is that the existing medicament structure for treating pterygium, an ophthalmic disease, is unitary, therefore, the present disclosure provides a use of a naphthylurea compound, which can treat pterygium by eye drops, filling the gap in this field and providing significant social and economic benefits.

The present disclosure provides a use of substance A in the manufacture of a medicament, wherein the substance A is a naphthylurea compound represented by formula I, a pharmaceutically acceptable salt thereof, a solvate thereof, a solvate of the pharmaceutically acceptable salt thereof, a crystal form thereof, or a tautomer thereof, and the medicament is used for the treatment of pterygium; 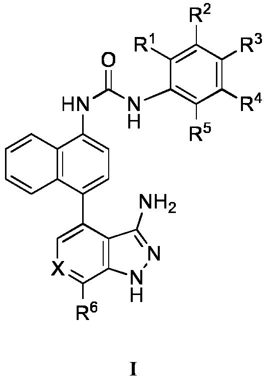
wherein R¹, R², R³, R⁴, and R⁵ are independently H, halogen, or C₁-C₆ alkyl;
X is CH or N;
R⁶ is H or -O-(CH₂)ₙ-Y;
Y is 5- to 6-membered heterocycloalkyl, and the heteroatom of the 5- to 6-membered heterocycloalkyl is N, and the number of heteroatoms is 1 to 2;
n is 2, 3, 4, or 5.

In a certain embodiment, in the naphthylurea compound represented by formula I, the pharmaceutically acceptable salt thereof, the solvate thereof, the solvate of the pharmaceutically acceptable salt thereof, the crystal form thereof, or the tautomer thereof, certain groups are defined as follows, and undefined groups are as defined in any of the preceding embodiments (this paragraph is simply referred to as "in a certain embodiment of the present disclosure"):
in R¹, R², R³, R⁴, or R⁵, the halogen may be fluorine, chlorine, bromine, or iodine, such as fluorine.

In a certain embodiment of the present disclosure, in R¹, R², R³, R⁴, or R⁵, the C₁-C₆ alkyl may be C₁-C₃ alkyl, such as methyl, ethyl, n-propyl, or isopropyl.

In a certain embodiment of the present disclosure, in Y, the 5- to 6-membered heterocycloalkyl may be 5- to 6-membered heterocycloalkyl containing 1 N, and another example is

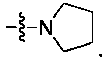

In a certain embodiment of the present disclosure, in R⁶, preferably, n is 2 or 3 (*e.g.,* 3).

In a certain embodiment of the present disclosure, preferably, 1 to 2 of R¹, R², R³, R⁴, and R⁵ are independently C₁-C₃ alkyl (e.g., methyl), and the rest are independently halogen or H.

In a certain embodiment of the present disclosure, preferably, 3 to 4 of R¹, R², R³, R⁴, and R⁵ are independently H, and the rest are independently halogen (e.g., fluorine) or C₁-C₃ alkyl (e.g., methyl).

In a certain embodiment of the present disclosure, X is CH, and R⁶ is -O-(CH₂)ₙ-Y.

In a certain embodiment of the present disclosure, X is N, and R⁶ is H.

In a certain embodiment of the present disclosure, 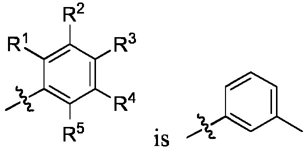 or 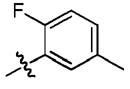 (e.g., 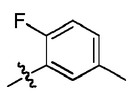 )

In a certain embodiment of the present disclosure, when X is N, and R⁶ is H, then 3 to 4 of R¹, R², R³, R⁴, and R⁵ are independently H, and the rest are independently halogen (e.g., fluorine) or C₁-C₃ alkyl (e.g., methyl);
when X is CH, and R⁶ is -O-(CH₂)ₙ-Y, then 1 of R¹, R², R³, R⁴, and R⁵ is halogen (e.g., fluorine), 1 of R¹, R², R³, R⁴, and R⁵ is C₁-C₃ alkyl (e.g., methyl), and the rest are H.

In a certain embodiment of the present disclosure, preferably, the naphthylurea compound represented by formula I is selected from any of the following compounds:

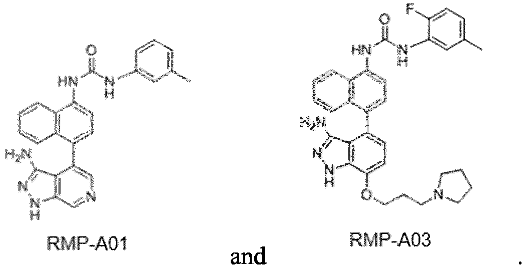

In the use, the dosage form of the medicament may be an eye drop.

In the eye drop, preferably, the substance A has a mass concentration of 10 to 30 mg/mL.

The eye drop refers to a sterile liquid preparation made from the medicament and a suitable ophthalmic pharmaceutical excipient. The eye drop can be divided into an aqueous solution eye drop, an oily solution eye drop, a suspension eye drop, or an emulsion eye drop.

The present disclosure further provides a pharmaceutical composition, and the pharmaceutical composition comprises the above substance A and an ophthalmic pharmaceutical excipient.

In the present disclosure, preferably, the pharmaceutical composition is a pharmaceutical composition for the treatment of pterygium.

The dosage form of the pharmaceutical composition may be an eye drop.

In the eye drop, preferably, the substance A has a mass concentration of 10 to 30 mg/mL (e.g., 20 mg/mL or 30 mg/mL).

The present disclosure further provides substance A for use in treating pterygium, and the substance A is as described above.

The present disclosure provides a method for treating pterygium in a patient in need thereof, comprising: administering to the patient in need thereof a therapeutically effective amount of the above substance A or the above pharmaceutical composition.

In the method, the "administering" may be in the form of eye drops.

In the method, the patient may be a mammal, such as a rabbit or a human.

In the method, the substance A or the pharmaceutical composition may be administered according to a conventional dose. Based on the substance A, a non-limiting example may range from 1 mg/eye to 3 mg/eye (single dose), such as 1 mg/eye.

In the method, the administration frequency of the substance A or the pharmaceutical composition may be four times a day.

Unless otherwise specified, the terms used in the present disclosure have the following meanings:
The term "pharmaceutically acceptable salt" refers to a salt prepared from the compound of the present disclosure and a relatively non-toxic and pharmaceutically acceptable acid or alkali. When the compound of the present disclosure contains relatively acidic functional groups, an alkali addition salt can be obtained by contacting a sufficient amount of pharmaceutically acceptable alkali with the neutral form of the compound in a pure solution or an appropriate inert solvent. The pharmaceutically acceptable alkali addition salt includes, but is not limited to, lithium salt, sodium salt, potassium salt, calcium salt, aluminum salt, magnesium salt, zinc salt, bismuth salt, ammonium salt, and diethanolamine salt. When the compound of the present disclosure contains relatively alkaline functional groups, an acid addition salt can be obtained by contacting a sufficient amount of pharmaceutically acceptable acid with the neutral form of the compound in a pure solution or an appropriate inert solvent. The pharmaceutically acceptable acid includes an inorganic acid, and the inorganic acid includes, but is not limited to, hydrochloric acid, hydrobromic acid, hydroiodic acid, nitric acid, carbonic acid, phosphoric acid, phosphorous acid, sulfuric acid, *etc.* The pharmaceutically acceptable acid includes an organic acid, and the organic acid includes, but is not limited to, acetic acid, propionic acid, oxalic acid, isobutyric acid, maleic acid, malonic acid, benzoic acid, succinic acid, suberic acid, trans-butenedioic acid, lactic acid, mandelic acid, phthalic acid, benzenesulfonic acid, p-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (*i.e.,* 4,4'-methylene-bis(3-hydroxy-2-naphthoic acid)), amino acid (*e.g.,* glutamic acid and arginine), *etc.* When the compound of the present disclosure contains relatively acidic functional groups and relatively alkaline functional groups, it can be converted into an alkali addition salt or an acid addition salt. For details, see Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining the compound of the present disclosure with a stoichiometric or non-stoichiometric amount of solvent. Solvent molecules in the solvate can exist in ordered or non-ordered arrangements. The solvent includes, but is not limited to, water, methanol, ethanol, *etc.*

The terms "pharmaceutically acceptable salt" and "solvate" in the term "solvate of pharmaceutically acceptable salt" are as described above, and the solvate of the pharmaceutically acceptable salt refers to a substance obtained by the following steps: 1. preparing a salt with a relatively non-toxic, pharmaceutically acceptable acid or base; 2. combining the salt with a stoichiometric or non-stoichiometric amount of a solvent. The "solvate of pharmaceutically acceptable salt" includes, but is not limited to, the hydrochloride monohydrate of the compound of the present disclosure.

The term "tautomer" refers to an isomer of a functional group resulting from a rapid movement of an atom between two positions in a molecule. For example, acetone and 1-propen-2-ol can be transformed into each other by rapid movement of a hydrogen atom on the oxygen and α-carbon.

The term "crystal form" refers to the strictly periodic arrangement of ions or molecules in a three-dimensional space in a certain manner, and has the law of periodic recurrence at a certain distance; due to the different periodic arrangements mentioned above, there may be many crystal forms, that is, polymorphism.

The term "alkyl" refers to a straight or branched chain alkyl group with a specified number of carbon atoms. Examples of alkyl groups include methyl, ethyl, *n*-propyl, isopropyl, *n*-butyl, *tert*-butyl*,* isobutyl, *sec*-butyl, *n*-pentyl, *n*-hexyl, and similar alkyl groups.

The term "heterocycloalkyl" refers to a saturated monocyclic group with a heteroatom.

The term "ophthalmic pharmaceutical excipient" refers to the excipient and additive used in the production of medicaments and preparation of prescriptions, and is all substances other than active ingredients included in pharmaceutical preparations. For details, see *Pharmacopoeia of the People's Republic of China* (2020 edition), or Handbook of Pharmaceutical Excipients (Raymond C Rowe, 2009 Sixth Edition).

The term "treatment" refers to therapeutic therapy. When referring to a specific condition, treatment refers to: (1) alleviating the disease or one or more biological manifestations of the condition, (2) interfering with (a) one or more points in the biological cascade that lead to or cause the condition or (b) one or more biological manifestations of the condition, (3) ameliorating one or more symptoms, effects, or side effects associated with the condition, or one or more symptoms, effects, or side effects associated with the condition or the treatment thereof, or (4) slowing the progression of the condition or one or more biological manifestations of the condition.

The term "therapeutically effective amount" refers to the amount of the compound that, when administered to a patient in need thereof, is sufficient to effectively treat the disease or condition described herein. The term "therapeutically effective amount" will vary based on the compound, the disease, and its severity, as well as the age of the patient to be treated, but can be adjusted as needed by those skilled in the art.

The term "patient" refers to any animal that will receive, or has already received, administration of the compound or the composition according to examples of the present disclosure, with mammals being preferred and humans being most preferred. The term "mammal" includes any mammal. Examples of mammals include, but are not limited to, cattle, horses, sheep, pigs, cats, dogs, mice, rats, rabbits, guinea pigs, monkeys, humans, *etc.,* with humans being most preferred.

The term "pterygium" is an ophthalmic disease.

On the basis of not violating common knowledge in the art, the above preferred conditions can be combined arbitrarily to obtain preferred examples of the present disclosure.

The reagents and raw materials used in the present disclosure are all commercially available.

The positive progressive effects of the present disclosure are that the naphthylurea compound of the present disclosure can be administered by eye drops to treat pterygium and other related ophthalmic diseases, filling the gap in this field and providing significant social and economic benefits.

### DETAILED DESCRIPTION OF THE PREFERRED EMBODIMENT

The present disclosure will be further described below with reference to examples, but the present disclosure is not therefore limited to the scope of the examples. Experimental methods without specific conditions in the following examples are selected according to conventional methods and conditions, or according to the commercial instructions.

The structures of the compounds used in the following examples are as follows:

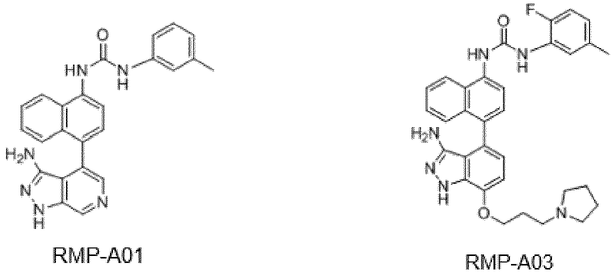

### Effect example 1

### 1. Experimental purpose

In this experiment, a pterygium model was induced in New Zealand white rabbits by NIH3T3 cells, and the compounds in the present disclosure were administered topically by eye drops, 3 times per day, and on day 13, the size of the pterygium was assessed by photographing the anterior segment of the animal's eye with a slit lamp. The inhibitory effect of the compounds of the present disclosure was explored on rabbit pterygium.

### 2. Experimental procedure

18 male New Zealand white rabbits were randomly divided into three groups according to their body weight: 6 rabbits in each group were injected with NIH3T3 cells and matrix membrane through bulbar conjunctiva.

The animals in each group, as well as the replacement animals, were modeled.

NIH3T3 cell revival: Immediately after receiving the cells, the cells were stored in a liquid nitrogen tank until revival; DMEM + 10% neonatal fetal bovine serum would expire after three months from preparation.

NIH3T3 cell culture: The revived cells were cultured in 5% CO₂, 37°C cell culture incubator, and the culture medium was changed twice a week, and cell passaging was performed when the cell density reached 70% to 75%.

NIH3T3 cell passaging: Cells were washed with PBS to ensure that the final cell density obtained was 8 × 10⁶ cells/mL. The obtained cells were mixed with the matrix membrane in an ice bath.

On Day 1, the animals were anesthetized with a combined intramuscular injection of xylazine (2 to 5 mg/kg) and ketamine (30 to 50 mg/kg). When the animal's corneal reflex disappeared, the animal was injected with 20 µL of a mixture of NIH3T3 cells and matrix membrane into the bulbar conjunctiva of both eyes of the animal.

Modeling was over.

Starting from Day 2, eye drops were administered three times a day, 50 µL/eye/time, until Day 12.

Administration method: After observing the administered preparation and confirming visual homogeneity, a micropipette was used to absorb 50 µL of vehicle or test sample, and dropped into the conjunctival sac of the animal. The animal's eyes were gently closed for several times to make the vehicle or test sample evenly distributed. To ensure homogeneity of the administered preparation, the preparation in the container could be turned upside down and, if necessary, vortexed appropriately.

Vehicle: 0.1% (w/v) poloxamer 188 and 2.4% (w/v) glycerol were dissolved in sterilized water for injection as a vehicle.

RMP-A01 eye drops test sample: 20 mg/mL RMP-A01 dissolved in the vehicle;
RMP-A03 eye drops test sample: 30 mg/mL RMP-A03 dissolved in the vehicle.

Examination items: On Day 13 after modeling, the anterior segment of the animal's eye was photographed with a slit lamp, and the size of pterygium was evaluated and the area was measured. The results are shown in Table 1.

**Table 1. Individual data of pterygium area in animals with RMP-A01 eye drops (mm²)**

| **Group** | **Animal number** | **Eye** | **DAY 13** |
|---|---|---|---|
| Vehicle group | 001 | Right eye | 14.83 |
| | 001 | Left eye | 22.09 |
| | 002 | Right eye | 21.25 |
| | 002 | Left eye | 20.62 |
| | 003 | Right eye | 12.72 |
| | 003 | Left eye | 18.55 |
| | 004 | Right eye | 13.57 |
| | 004 | Left eye | 28.49 |
| | 005 | Right eye | 24.35 |
| | 005 | Left eye | 24.06 |
| | 006 | Right eye | 24.47 |
| | 006 | Left eye | 12.94 |
| | Average value | | 19.83 |
| RMP-A01 eye drops | 001 | Right eye | 15.83 |
| | 001 | Left eye | 20.53 |
| | 002 | Right eye | 10.64 |
| | 002 | Left eye | 17.36 |
| | 003 | Right eye | 9.81 |
| | 003 | Left eye | 9.45 |
| | 004 | Right eye | 23.76 |
| | 004 | Left eye | 19.36 |
| | 005 | Right eye | 11.86 |
| | 005 | Left eye | 6.78 |
| | 006 | Right eye | 8.76 |
| | 006 | Left eye | 18.74 |
| | Average value | | 14.41 |
| RMP-A03 eye drops | 001 | Right eye | 10.28 |
| | 001 | Left eye | 12.1 |
| | 002 | Right eye | 13.25 |
| | 002 | Left eye | 13.02 |
| | 003 | Right eye | 13.84 |
| | 003 | Left eye | 13.87 |
| | 004 | Right eye | 15.35 |
| | 004 | Left eye | 12.65 |
| | 005 | Right eye | 15.7 |
| | 005 | Left eye | 8.88 |
| | 006 | Right eye | 15.25 |
| | 006 | Left eye | 12.68 |
| | Average value | | 13.07 |

On Day 13, both RMP-A01 eye drops and RMP-A03 eye drops test sample administration groups showed a decrease in pterygium area compared to the vehicle control group.

### 3. Experimental conclusion

Under the experimental conditions, NIH3T3 can successfully induce the rabbit pterygium model.

Under the experimental conditions, the compound of the present disclosure tends to reduce the pterygium area under the above experimental administration conditions.

In summary, the novel compound represented by formula I disclosed in the present disclosure provides a novel drug possibility for the clinical treatment of pterygium diseases.

Although specific embodiments of the present disclosure have been described above, those skilled in the art should understand that these are only examples, and various changes or modifications can be made to these embodiments without departing from the principles and essence of the present disclosure. Therefore, the scope of protection for the present disclosure is defined by the appended claims.

## Claims

1. A use of substance A in the manufacture of a medicament, wherein the substance A is a naphthylurea compound represented by formula I, a pharmaceutically acceptable salt thereof, a solvate thereof, a solvate of the pharmaceutically acceptable salt thereof, a crystal form thereof, or a tautomer thereof, and the medicament is used for the treatment of pterygium; 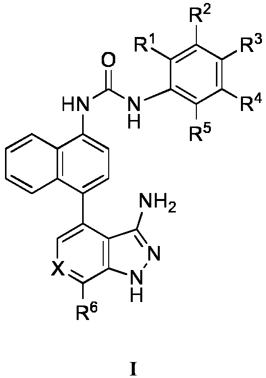
wherein R¹, R², R³, R⁴, and R⁵ are independently H, halogen, or C₁-C₆ alkyl;
X is CH or N;
R⁶ is H or -O-(CH₂)ₙ-Y;
Y is 5- to 6-membered heterocycloalkyl, and the heteroatom of the 5- to 6-membered heterocycloalkyl is N, and the number of heteroatoms is 1 to 2;
n is 2, 3, 4, or 5.

2. The use of substance A in the manufacture of the medicament according to claim 1, wherein in R¹, R², R³, R⁴, or R⁵, the halogen is fluorine, chlorine, bromine, or iodine;
and/or, in R¹, R², R³, R⁴, or R⁵, the C₁-C₆ alkyl is C₁-C₃ alkyl;
and/or, in Y, the 5- to 6-membered heterocycloalkyl is 5- to 6-membered heterocycloalkyl containing 1 N;
and/or, in R⁶, n is 2 or 3.

3. The use of substance A in the manufacture of the medicament according to claim 2, wherein in R¹, R², R³, R⁴, or R⁵, the halogen is fluorine;
and/or, in R¹, R², R³, R⁴, or R⁵, the C₁-C₆ alkyl is methyl, ethyl, n-propyl, or isopropyl;
and/or, in Y, the 5- to 6-membered heterocycloalkyl is 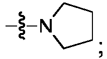
and/or, in R⁶, n is 3.

4. The use of substance A in the manufacture of the medicament according to claim 1, wherein 1 to 2 of R¹, R², R³, R⁴, and R⁵ are independently C₁-C₃ alkyl, and the rest are independently halogen or H, wherein the C₁-C₃ alkyl is preferably methyl;
and/or, 3 to 4 of R¹, R², R³, R⁴, and R⁵ are independently H, and the rest are independently halogen or C₁-C₃ alkyl; wherein the halogen is preferably fluorine, and the C₁-C₃ alkyl is preferably methyl.

5. The use of substance A in the manufacture of the medicament according to claim 1, wherein X is CH, and R⁶ is -O-(CH₂)ₙ-Y; or, X is N, and R₆ is H;
and/or, 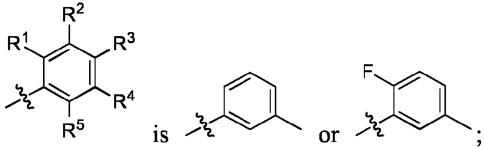 more preferably, 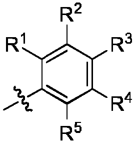 is

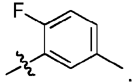

6. The use of substance A in the manufacture of the medicament according to claim 1, wherein when X is N, and R⁶ is H, then 3 to 4 of R¹, R², R³, R⁴, and R⁵ are independently H, and the rest are independently halogen or C₁-C₃ alkyl; wherein the halogen is preferably fluorine, and the C₁-C₃ alkyl is preferably methyl;
when X is CH, and R⁶ is -O-(CH₂)ₙ-Y, then 1 of R¹, R², R³, R⁴, and R⁵ is halogen, 1 of R¹, R², R³, R⁴, and R⁵ is C₁-C₃ alkyl, and the rest are H; wherein the halogen is preferably fluorine, and the C₁-C₃ alkyl is preferably methyl.

7. The use of substance A in the manufacture of the medicament according to claim 1, wherein the naphthylurea compound represented by formula I is selected from any of the following compounds:

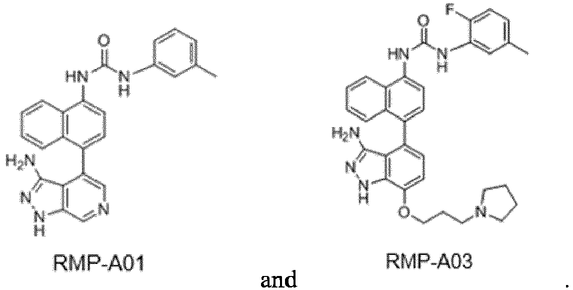

8. The use of substance A in the manufacture of the medicament according to claim 1, wherein the dosage form of the medicament is an eye drop; the eye drop is preferably an aqueous solution eye drop, an oily solution eye drop, a suspension eye drop, or an emulsion eye drop;
in the eye drop, preferably, the substance A has a mass concentration of 10 to 30 mg/mL.

9. A pharmaceutical composition, wherein the pharmaceutical composition comprises the substance A according to any one of claims 1 to 8 and an ophthalmic pharmaceutical excipient.

10. The pharmaceutical composition according to claim 9, wherein the dosage form of the pharmaceutical composition is an eye drop; in the eye drop, preferably, the substance A has a mass concentration of 10 to 30 mg/mL, and further the mass concentration is 20 mg/mL or 30 mg/mL;
and/or, the pharmaceutical composition is a pharmaceutical composition for the treatment of pterygium.
